# EUROPEAN PATENT SPECIFICATION

(11) **EP 4 277 535 B1**
(45) Date of publication and mention of the grant of the patent: **06.08.2025**
(21) Application number: 22701319.0
(22) Date of filing: 12.01.2022
(51) Int. Cl.: A61B 8/12, A61B 8/00

(54) **FLEXIBLE ADHESIVE-FILLED DISTAL REGION FOR INTRALUMINAL IMAGING DEVICE**
FLEXIBLER, MIT KLEBSTOFF GEFÜLLTER, DISTALER BEREICH FÜR EINE INTRALUMINALE BILDGEBUNGSVORRICHTUNG
RÉGION DISTALE REMPLIE D'ADHÉSIF FLEXIBLE POUR DISPOSITIF D'IMAGERIE INTRALUMINALE

(30) Priority: 15.01.2021 US 202163137923 P
(43) Date of publication of application: 22.11.2023
(73) Proprietor: Koninklijke Philips N.V., 5656 AG Eindhoven (NL); Philips Image Guided Therapy Corporation, San Diego CA 92130 (US)
(72) Inventor: LEBLANC, Christopher, 5656 AG Eindhoven (NL); BADERTSCHER, Ryan, 5656 AG Eindhoven (NL); GARCIA, Brittany, 5656 AG Eindhoven (NL); RANGARAJAN, Swathi, 5656 AG Eindhoven (NL); SASAMINE, Kazuo, 5656 AG Eindhoven (NL)
(74) Representative: Philips Intellectual Property & Standards
(86) International application number: PCT/EP2022/050495
(87) International publication number: WO 2022/152724

(56) References cited:
- US-A1- 2005 215 942
- US-A1- 2016 058 382
- US-A1- 2020 297 306

## Description

### TECHNICAL FIELD

The present disclosure relates generally to intraluminal imaging devices and, in particular, to intraluminal imaging devices comprising flexible elongate members with regions having different flexibility. For example, intravascular ultrasound (IVUS) imaging catheter includes a flexible distal region that is filled with adhesive.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Phased array (also known as synthetic-aperture) IVUS catheters are a type of IVUS device commonly used today. Phased array IVUS catheters carry a scanner assembly that includes an array of ultrasound transducers positioned and distributed around its perimeter or circumference along with one or more integrated circuit controller chips mounted adjacent to the transducer array. The controllers select individual acoustic elements (or groups of elements) for transmitting a pulse of acoustic energy and for receiving the ultrasound echo signal corresponding to the transmitted ultrasound energy. By stepping through a sequence of transmit-receive pairs, the phased array IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma and without a need for an additional housing between the rotating element and the vessel lumen.

IVUS catheters must be stiff enough to be pushable, so that a clinician can advance them through the tortuous pathways of human vasculature. However, to facilitate navigation through these tortuous pathways, the catheters must also be flexible. It can be challenging to design IVUS catheters that meet both of these requirements simultaneously.

For example, document US2016058382 A1 discloses guide wires that include a distal coil filled with a flexible adhesive.

### SUMMARY

Disclosed herein is an intraluminal imaging device (e.g., an intravascular ultrasound or IVUS imaging device) advantageously providing both pushability and flexibility for navigation through vasculature. The device includes a flexible elongate member (e.g., a catheter) with a proximal portion and a distal portion, and an imaging assembly disposed at the distal portion for obtaining intraluminal image data. The distal portion of the flexible elongate member comprises a flexible region, proximal to the imaging assembly, that is more flexible than the proximal portion, and is filled with an adhesive whose hardness helps to define the flexibility of the region, and is surrounded by an outer polymer layer. The outer polymer layer includes an opening for injecting the adhesive, after which the opening is filled with a plug of material (e.g., a second adhesive). The proximal region of the flexible elongate member includes a polymer-coated metal shaft that is cut in a spiral configuration to provide both flexibility and pushability. A guidewire lumen extends from the proximal portion the distal portion of the flexible elongate member through a guidewire lumen defined by a polymer-coated braided metal layer.

One general aspect of the intraluminal imaging device includes a flexible elongate member configured to be positioned within a body lumen of a patient, where the flexible elongate member includes a proximal portion and a distal portion; and an imaging assembly disposed at the distal portion of the flexible elongate member and configured to obtain intraluminal image data while positioned within the body lumen. The distal portion of the flexible elongate member includes a region proximal to the imaging assembly, where the region includes a first hardness that is less than a second hardness of the proximal portion of the flexible elongate member such that the region is more flexible than the proximal portion. The region includes: an inner member; a first adhesive configured to provide the first hardness, where the first adhesive surrounds the inner member; a communication line configured to carry signals associated with the imaging assembly, where the communication line is embedded within the first adhesive; and an outer polymer layer surrounding the first adhesive.

Implementations may include one or more of the following features. In some embodiments, the outer polymer layer includes an opening filled with a second adhesive. In some embodiments, the second adhesive is fluorescent under ultraviolet (UV) light. In some embodiments, the flexible elongate member includes an outer member proximal to the region, where the outer member includes a metal shaft and a polymer coating surrounding the metal shaft. In some embodiments, the metal shaft is cut in a spiral configuration. In some embodiments, the inner member and the communication line extend within the outer member. In some embodiments, the flexible elongate member includes a catheter, and where the inner member defines a guidewire lumen. In some embodiments, a distal portion of the inner member and a proximal portion the imaging assembly are positioned within the polymer tube. In some embodiments, the imaging assembly includes: a flexible substrate; and a plurality of integrated circuits disposed on the flexible substrate, where the polymer tube extends over the plurality of integrated circuits. In some embodiments, the device further including a third adhesive positioned within the polymer tube. In some embodiments, the communication line includes an electrical conductor; where the imaging assembly includes: a support member; a flexible substrate positioned around the support member, where the electrical conductor is coupled to a proximal portion of the flexible substrate; and insulation positioned around the support member and configured to prevent contact between the proximal portion of the flexible substrate and the support member. In some embodiments, the inner member defines a guidewire lumen extending from the proximal portion of the flexible elongate member to the distal portion of the flexible elongate member. In some embodiments, the outer polymer layer includes an outer surface of the flexible elongate member in the region. In some embodiments, the inner member includes: a first polymer layer defining a guidewire lumen; a wire braid surrounding the first polymer layer; and a second polymer layer surrounding the first polymer layer. In some embodiments, the flexible elongate member includes a catheter, where the body lumen includes a blood vessel, where the imaging assembly includes a circumferential array of acoustic elements, and where the intraluminal image data includes intravascular ultrasound (IVUS) image data.

One general aspect includes an intravascular ultrasound (IVUS) imaging device. The device includes a catheter configured to configured to be positioned within a blood vessel of a patient, where the catheter includes a proximal portion and a distal portion; and a circumferential array of acoustic elements disposed at the distal portion of the catheter and configured to obtain IVUS image data while positioned within the blood vessel, where the distal portion of the catheter includes a region proximal to the circumferential array of acoustic elements, where the region includes a first hardness that is less than a second hardness of the proximal portion of the catheter such that the region is more flexible than the proximal portion, where the region includes: an inner member including: a first polymer layer defining a guidewire lumen; a wire braid surrounding the first polymer layer; and a second polymer layer surrounding the first polymer layer; an adhesive configured to provide the first hardness, where the adhesive surrounds the inner member; a plurality of electrical conductors in communication with the circumferential array of acoustic elements, where the plurality of electrical conductors are embedded within the adhesive; and a third polymer layer surrounding the adhesive and forming an outer surface of catheter in the region.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
**Fig. 1A** is a diagrammatic schematic view of an intraluminal imaging system, according to aspects of the present disclosure.
**Fig. 1B** is a schematic diagram of a processor circuit, according to embodiments of the present disclosure.
**Fig. 2** is a diagrammatic view of the top of a scanner assembly in a flat configuration, according to aspects of the present disclosure.
**Fig. 3** is a diagrammatic perspective view of the scanner assembly shown in Fig. 2 in a rolled configuration around a support member, according to aspects of the present disclosure.
**Fig. 4** is a diagrammatic cross-sectional side view of a scanner assembly in a rolled configuration around a support member, according to aspects of the present disclosure.
**Fig. 5** is a diagrammatic side view of an intraluminal imaging device, in accordance with at least one embodiment of the present disclosure.
**Fig. 6** is a diagrammatic, cross-sectional side view of an example flexible elongate member, in accordance with at least one embodiment of the present disclosure.
**Fig. 7** is a diagrammatic, cross-sectional side view of a distal portion of an example flexible elongate member, in accordance with at least one embodiment of the present disclosure.
**Fig. 8** is a is a diagrammatic, cross-sectional perspective view of a distal portion of an example flexible elongate member, in accordance with at least one embodiment of the present disclosure.
**Fig. 9** is a is a diagrammatic, cross-sectional side view of a scanner assembly of an example flexible elongate member, in accordance with at least one embodiment of the present disclosure.
**Fig. 10** is a diagrammatic, cross-sectional perspective view of cut plane 10-10 from Fig. 6, in accordance with at least one embodiment of the present disclosure.
**Fig. 11** is a diagrammatic, cross-sectional view of cut plane 10-10 from Fig. 6, in accordance with at least one embodiment of the present disclosure.
**Fig. 12** is a diagrammatic, cross-sectional perspective view of cut plane 12-12 of Fig. 6, in accordance with at least one embodiment of the present disclosure.
**Fig. 13** is a diagrammatic, cross-sectional perspective view of cut plane 13-13 from Fig. 7, in accordance with at least one embodiment of the present disclosure.
**Fig. 14** is a diagrammatic, cross-sectional perspective view of cut plane 14-14 of from Fig. 7, in accordance with at least one embodiment of the present disclosure.
**Fig. 15** is a diagrammatic, cross-sectional perspective view of cut plane 15-15 from Fig. 7, in accordance with at least one embodiment of the present disclosure.
**Fig. 16** is a perspective view of an interface region between regions of an example flexible elongate member, in accordance with at least one embodiment of the present disclosure.
**Fig. 17** is a diagrammatic, cross-sectional side view of the regions from Fig. 16, in accordance with at least one embodiment of the present disclosure.
**Fig. 18** is a perspective view of an example flexible elongate member, in accordance with at least one embodiment of the present disclosure.

### DETAILED DESCRIPTION

Disclosed herein is an intraluminal imaging device with stiff portions to facilitate pushing, flexible portions to facilitate navigation, and rigid portions to facilitate imaging within body lumens such as human blood vessels. The intraluminal imaging device comprises a flexible elongate member (e.g., a catheter) for use within a body lumen (e.g., a blood vessel) of a patient. The flexible elongate member has a proximal portion and a distal portion, and an imaging assembly located at the distal portion and configured to obtain intraluminal image data (e.g., IVUS image data). The distal portion of the flexible elongate member comprises a region, proximal to the imaging assembly, that is more flexible than the proximal portion. The region includes an inner member that comprises a guidewire lumen and that is surrounded by an adhesive whose hardness or durometer helps to define the flexibility of the region. The region also includes communication lines (e.g., wires, optical fibers, etc.) embedded within the adhesive, to carry signals associated with the imaging assembly. The region also includes an outer polymer layer surrounding the first adhesive, whose outer diameter defines the outer diameter of the region. The outer polymer layer includes an opening filled with a plug of material (e.g., a second adhesive) that may be fluorescent under ultraviolet (UV) light to facilitate inspection of the plug.

The flexible elongate member also includes, proximal to the adhesive-filled region, an outer member comprising a metal shaft surrounded by a polymer coating. The metal shaft is cut in a spiral configuration to provide flexibility, while the inner member and the communication lines extend through a lumen within the outer member.

The imaging assembly of the device includes a circumferential array of acoustic elements for gathering intravascular ultrasound (IVUS) image data. A distal portion of the inner member and a proximal portion of the imaging assembly are positioned within a polymer tube, which extends over a portion of a flexible substrate of the imaging assembly (e.g., over one or more weld legs that join the communication lines to the flexible substrate and/or over a plurality of integrated circuits on the flexible substrate). The flexible substrate is wrapped around a support member (e.g., a metallic ferrule). A third adhesive may be positioned within the polymer tube, and an insulating material (which may also be an adhesive) is positioned around the support member and configured to prevent electrical contact (e.g., shorting) between the proximal portion of the flexible substrate and the support member.

The inner member comprises a guidewire lumen extending from the proximal portion the distal portion of the flexible elongate member. The wall of the guidewire lumen is defined by a first polymer layer, which is surrounded by a wire braid layer, which is surrounded by a second polymer layer. The second polymer layer defines the outer surface of the inner member.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

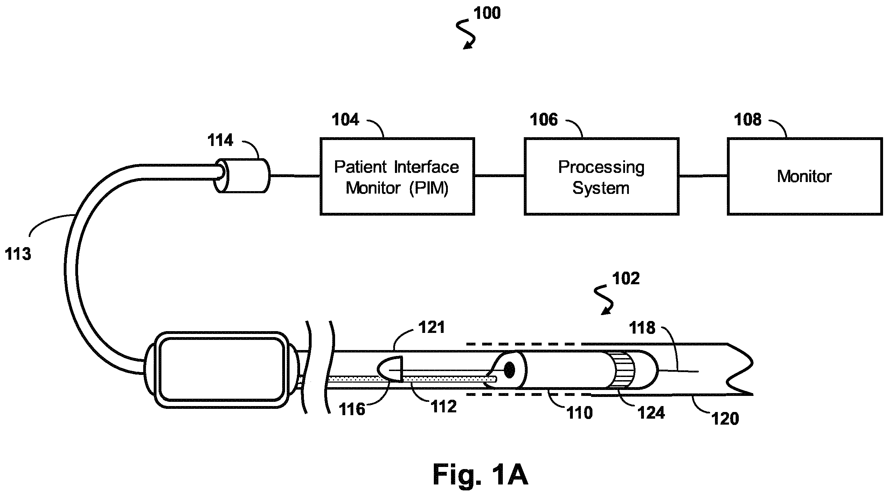

**Fig. 1A** is a diagrammatic schematic view of an ultrasound imaging system 100, according to aspects of the present disclosure. The ultrasound imaging system 100 can be an intraluminal imaging system. In some instances, the system 100 can be an intravascular ultrasound (IVUS) imaging system. The system 100 may include an intraluminal imaging device 102 such as a catheter, guide wire, or guide catheter, a patient interface module (PIM) 104, a processing system or console 106, and a monitor 108. The intraluminal imaging device 102 can be an ultrasound imaging device. In some instances, the device 102 can be IVUS imaging device, such as a solid-state IVUS device.

At a high level, the IVUS device 102 emits ultrasonic energy, or ultrasound signals, from a transducer array 124 included in scanner assembly 110 mounted near a distal end of the catheter device. The ultrasonic energy is reflected by tissue structures in the medium, such as a vessel 120, or another body lumen surrounding the scanner assembly 110, and the ultrasound echo signals are received by the transducer array 124. In that regard, the device 102 can be sized, shaped, or otherwise configured to be positioned within the body lumen of a patient. The PIM 104 transfers the received echo signals to the console or computer 106 where the ultrasound image (possibly including flow information) is reconstructed and displayed on the monitor 108. The processing system 106 can include a processor and a memory. The processing system 106 can be operable to facilitate the features of the IVUS imaging system 100 described herein. For example, the processing system 106 can execute computer readable instructions stored on the non-transitory tangible computer readable medium.

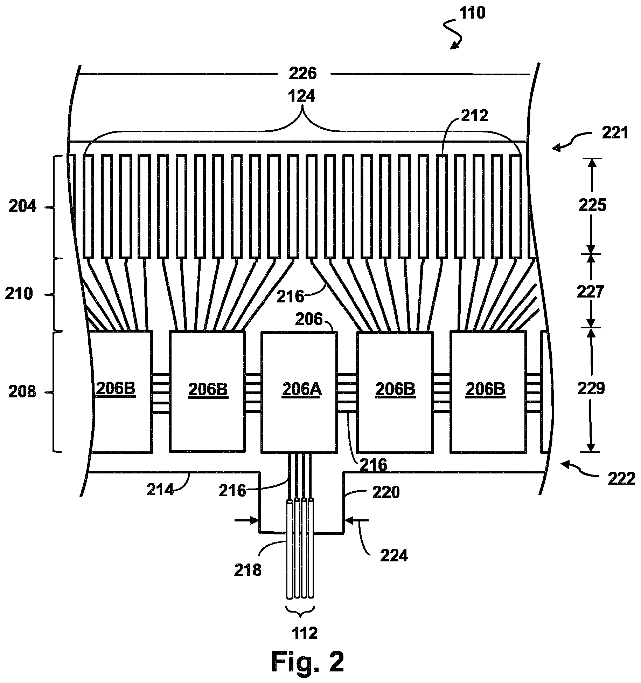

The PIM 104 facilitates communication of signals between the processing system 106 and the scanner assembly 110 included in the IVUS device 102. This communication includes the steps of: (1) providing commands to integrated circuit controller chip(s) 206A, 206B, illustrated in Fig. 2, included in the scanner assembly 110 to select the particular transducer array element(s), or acoustic element(s), to be used for transmit and receive, (2) providing the transmit trigger signals to the integrated circuit controller chip(s) 206A, 206B included in the scanner assembly 110 to activate the transmitter circuitry to generate an electrical pulse to excite the selected transducer array element(s) 212, and/or (3) accepting amplified echo signals received from the selected transducer array element(s) 212 via amplifiers included on the integrated circuit controller chip(s) 206 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The processing system 106 receives the echo data from the scanner assembly 110 by way of the PIM 104 and processes the data to reconstruct an image of the tissue structures in the medium surrounding the scanner assembly 110. The console 106 outputs image data such that an image of the vessel 120, such as a cross-sectional image of the vessel 120, is displayed on the monitor 108. Vessel 120 may represent fluid filled or surrounded structures, both natural and man-made. The vessel 120 may be within a body of a patient. The vessel 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

In some embodiments, the IVUS device includes some features similar to traditional solid-state IVUS catheters, such as the EagleEye^{®} catheter available from Koninklijke Philips N.V. and those disclosed in U.S. Patent No. 7,846,101**.** For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors 218 (Fig. 2). Further, in some embodiments, the IVUS device 102 includes a plurality of transmission line bundles each comprising a plurality of conductors of varying size (e.g., gauge), insulation, and/or other structural and electrical characteristics. It is understood that any suitable gauge wire can be used for the conductors 218. In an embodiment, the cable 112 can include a four-conductor transmission line arrangement with, e.g., 41 AWG gauge wires. In an embodiment, the cable 112 can include a seven-conductor transmission line arrangement utilizing, e.g., 44 AWG gauge wires. In some embodiments, 43 AWG gauge wires can be used.

The transmission line bundle 112 passes through or connects to a cable 113 that terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

In an embodiment, the processing system 106 generates flow data by processing the echo signals from the IVUS device 102 into Doppler power or velocity information. The processing system 106 may also generate B-mode data by applying envelope detection and logarithmic compression on the conditioned echo signals. The processing system 106 can further generate images in various views, such as 2D and/or 3D views, based on the flow data or the B-mode data. The processing system 106 can also perform various analyses and/or assessments. For example, the processing system 106 can apply virtual histology (VH) techniques, for example, to analyze or assess plaques within a vessel (e.g., the vessel 120). The images can be generated to display a reconstructed color-coded tissue map of plaque composition superimposed on a cross-sectional view of the vessel.

In an embodiment, the processing system 106 can apply a blood flow detection algorithm to determine the movement of blood flow, for example, by acquiring image data of a target region (e.g., the vessel 120) repeatedly and determining the movement of the blood flow from the image data. The blood flow detection algorithm operates based on the principle that signals measured from vascular tissue are relatively static from acquisition to acquisition, whereas signals measured from blood flow vary at a characteristic rate corresponding to the flow rate. As such, the blood flow detection algorithm may determine movements of blood flow based on variations in signals measured from the target region between repeated acquisitions. To acquire the image data repeatedly, the processing system 106 may control to the device 102 to transmit repeated pulses on the same aperture.

While the present disclosure describes embodiments related to intravascular ultrasound (IVUS) imaging using an intravascular catheter or guidewire, it is understood that one or more aspects of the present disclosure can be implemented in any suitable ultrasound imaging system, including a synthetic aperture ultrasound imaging system, a phased array ultrasound imaging system, or any other array-based ultrasound imaging system. For example, aspects of the present disclosure can be implemented in intraluminal ultrasound imaging systems using an intracardiac (ICE) echocardiography catheter and/or a transesophageal echocardiography (TEE) probe, and/or external ultrasound imaging system using an ultrasound probe configured for imaging while positioned adjacent to and/or in contact with the patient's skin. The ultrasound imaging device can be a transthoracic echocardiography (TTE) imaging device in some embodiments.

An ultrasound transducer array of the ultrasound imaging device includes an array of acoustic elements configured to emit ultrasound energy and receive echoes corresponding to the emitted ultrasound energy. In some instances, the array may include any number of ultrasound transducer elements. For example, the array can include between 2 acoustic elements and 10000 acoustic elements, including values such as 2 acoustic elements, 4 acoustic elements, acoustic elements, 64 acoustic elements, 128 acoustic elements, 500 acoustic elements, 812 acoustic elements, 3000 acoustic elements, 9000 acoustic elements, and/or other values both larger and smaller. In some instances, the transducer elements of the array may be arranged in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of transducer elements (e.g., one or more rows, one or more columns, and/or one or more orientations) can be uniformly or independently controlled and activated. The array can be configured to obtain one-dimensional, two-dimensional, and/or three-dimensional images of patient anatomy.

The ultrasound transducer elements may include piezoelectric/piezoresistive elements, piezoelectric micromachined ultrasound transducer (PMUT) elements, capacitive micromachined ultrasound transducer (CMUT) elements, and/or any other suitable type of ultrasound transducer elements. The ultrasound transducer elements of the array are in communication with (e.g., electrically coupled to) electronic circuitry. For example, the electronic circuitry can include one or more transducer control logic dies. The electronic circuitry can include one or more integrated circuits (IC), such as application specific integrated circuits (ASICs). In some embodiments, one or more of the ICs can include a microbeamformer (µBF). In other embodiments, one or more of the ICs includes a multiplexer circuit (MUX).

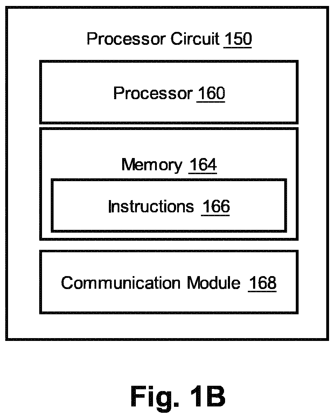

**Fig. 1B** is a schematic diagram of a processor circuit 150, according to embodiments of the present disclosure. The processor circuit 150 may be implemented in the processing system 106 and/or the imaging device 102 of Fig. 1A. As shown, the processor circuit 150 may include a processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 164 includes a non-transitory computer-readable medium. The memory 164 may store instructions 166. The instructions 166 may include instructions that, when executed by the processor 160, cause the processor 160 to perform the operations described herein with reference to the processing system 106 and/or the imaging device 102 (Fig. 1A). Instructions 166 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 168 can include any electronic circuitry and/or logic circuitry to facilitate direct or indirect communication of data between the processor circuit 150, the imaging device 102, and/or the display 108. In that regard, the communication module 168 can be an input/output (I/O) device. In some instances, the communication module 168 facilitates direct or indirect communication between various elements of the processor circuit 150 and/or the processing system 106 (Fig. 1A).

**Fig. 2** is a diagrammatic top view of a portion of a flexible assembly 200, according to aspects of the present disclosure. The flexible assembly 200 includes a transducer array 124 formed in a transducer region 204 and transducer control logic dies 206 (including dies 206A and 206B) formed in a control region 208, with a transition region 210 disposed therebetween.

The transducer control logic dies 206 are mounted on a flexible substrate 214 into which the transducers 212 have been previously integrated. The flexible substrate 214 is shown in a flat configuration in Fig. 2. Though six control logic dies 206 are shown in Fig. 2, any number of control logic dies 206 may be used. For example, one, two, three, four, five, six, seven, eight, nine, ten, or more control logic dies 206 may be used.

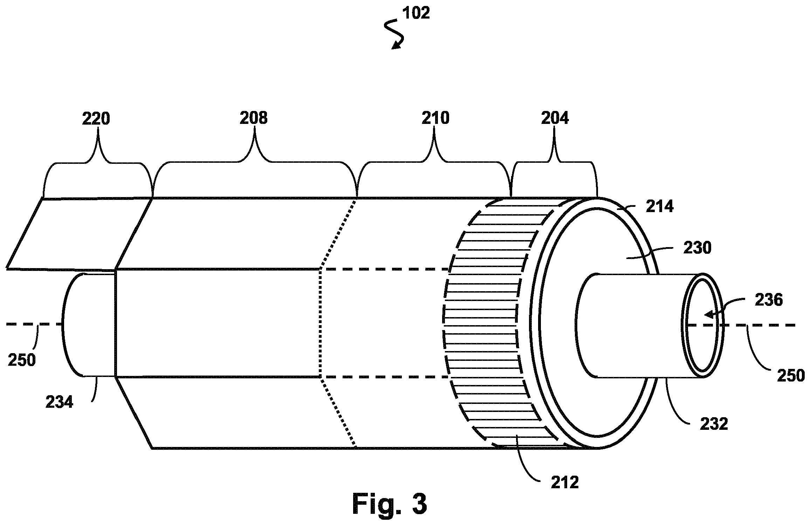

The flexible substrate 214, on which the transducer control logic dies 206 and the transducers 212 are mounted, provides structural support and interconnects for electrical coupling. The flexible substrate 214 may be constructed to include a film layer of a flexible polyimide material such as KAPTON^{™} (trademark of DuPont). Other suitable materials include polyester films, polyimide films, polyethylene napthalate films, or polyetherimide films, liquid crystal polymer, other flexible printed semiconductor substrates as well as products such as Upilex^{®} (registered trademark of Ube Industries) and TEFLON^{®} (registered trademark of E.I. du Pont). In the flat configuration illustrated in Fig. 2, the flexible substrate 214 has a generally rectangular shape. As shown and described herein, the flexible substrate 214 is configured to be wrapped around a support member 230 (Fig. 3) in some instances. Therefore, the thickness of the film layer of the flexible substrate 214 is generally related to the degree of curvature in the final assembled flexible assembly 200. In some embodiments, the film layer is between 5 µm and 100 µm, with some particular embodiments being between 5 µm and 25.1 µm, e.g., 6 µm.

The transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for the transmission line bundle 112, which may serve as an electrical communication bus between a processing system, e.g., processing system 106, and the flexible assembly 200. Accordingly, the master control circuit may include control logic that decodes control signals received over the transmission line bundle 112, transmits control responses over the transmission line bundle 112, amplifies echo signals, and/or transmits the echo signals over the transmission line bundle 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

To electrically interconnect the control logic dies 206 and the transducers 212, in an embodiment, the flexible substrate 214 includes conductive traces 216 formed in the film layer that carry signals between the control logic dies 206 and the transducers 212. In particular, the conductive traces 216 providing communication between the control logic dies 206 and the transducers 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of the transmission line bundle 112 when the conductors 218 of the transmission line bundle 112 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 µm. For example, in an embodiment, 5 µm conductive traces 216 are separated by 5 µm of space. The width of a conductive trace 216 on the flexible substrate may be further determined by the width of the conductor 218 to be coupled to the trace/pad.

The flexible substrate 214 can include a conductor interface 220 in some embodiments. The conductor interface 220 can be a location of the flexible substrate 214 where the conductors 218 of the transmission line bundle 112 are coupled to the flexible substrate 214. For example, the bare conductors of the transmission line bundle 112 are electrically coupled to the flexible substrate 214 at the conductor interface 220. The conductor interface 220 can be a tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN^{®}), polyether ether ketone (PEEK), nylon, Liquid Crystal Polymer (LCP), and/or other suitable materials.

**Fig. 3** illustrates a perspective view of the device 102 with the scanner assembly 200 in a rolled configuration. In some instances, the assembly 200 is transitioned from a flat configuration (Fig. 2) to a rolled or more cylindrical configuration (Fig. 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE".

In some embodiments, the transducer elements 212 and/or the controllers 206 can be positioned in in an annular configuration, such as a circular configuration or in a polygon configuration, around a longitudinal axis 250 of a support member 230. It will be understood that the longitudinal axis 250 of the support member 230 may also be referred to as the longitudinal axis of the scanner assembly 200, the flexible elongate member 121, and/or the device 102. For example, a cross-sectional profile of the imaging assembly 200 at the transducer elements 212 and/or the controllers 206 can be a circle or a polygon. Any suitable annular polygon shape can be implemented, such as a based on the number of controllers/transducers, flexibility of the controllers/transducers, etc., including a pentagon, hexagon, heptagon, octagon, nonagon, decagon, etc. In some examples, the plurality of transducer controllers 206 may be used for controlling the plurality of ultrasound transducer elements 212 to obtain imaging data associated with the vessel 120.

The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014, ('220 Application). The support member 230 can be a ferrule having a distal flange or portion 232 and a proximal flange or portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process.

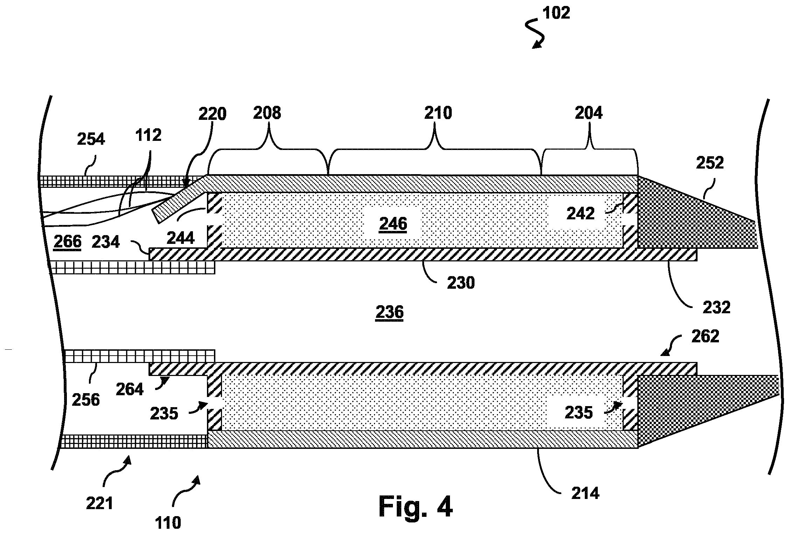

**Fig. 4** is a diagrammatic cross-sectional side view of a distal portion of the intraluminal imaging device 102, including the flexible substrate 214 and the support member 230, according to aspects of the present disclosure. The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014. The support member 230 can be a ferrule having a distal portion 262 and a proximal portion 264. The support member 230 can define a lumen 236 extending along the longitudinal axis LA. The lumen 236 is in communication with the entry/exit port 116 and is sized and shaped to receive the guide wire 118 (Fig. 1A). The support member 230 can be manufactured according to any suitable process. For example, the support member 230 can be machined and/or electrochemically machined or laser milled, such as by removing material from a blank to shape the support member 230, or molded, such as by an injection molding process. In some embodiments, the support member 230 may be integrally formed as a unitary structure, while in other embodiments the support member 230 may be formed of different components, such as a ferrule and stands 242, 244, that are fixedly coupled to one another. In some cases, the support member 230 and/or one or more components thereof may be completely integrated with inner member 256. In some cases, the inner member 256 and the support member 230 may be joined as one, e.g., in the case of a polymer support member.

Stands 242, 244 that extend vertically are provided at the distal and proximal portions 262, 264, respectively, of the support member 230. The stands 242, 244 elevate and support the distal and proximal portions of the flexible substrate 214. In that regard, portions of the flexible substrate 214, such as the transducer portion or region 204, can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244 and can also have special features for rotational alignment as well as control chip placement and connection. To improve acoustic performance, any cavities between the flexible substrate 214 and the surface of the support member 230 are filled with a backing material 246. The liquid backing material 246 can be introduced between the flexible substrate 214 and the support member 230 via passageways 235 in the stands 242, 244. In some embodiments, suction can be applied via the passageways 235 of one of the stands 242, 244, while the liquid backing material 246 is fed between the flexible substrate 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 262 and/or increased diameter proximal portion 264 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can include a flexible elongate member. The proximal inner member 256 can be received within a proximal flange 234. The proximal outer member 254 abuts and is in contact with the flexible substrate 214. A distal member 252 is coupled to the distal portion 262 of the support member 230. For example, the distal member 252 is positioned around the distal flange 232. The distal member 252 can abut and be in contact with the flexible substrate 214 and the stand 242. The distal member 252 can be the distal-most component of the intraluminal imaging device 102.

One or more adhesives can be disposed between various components at the distal portion of the intraluminal imaging device 102. For example, one or more of the flexible substrate 214, the support member 230, the distal member 252, the proximal inner member 256, and/or the proximal outer member 254 can be coupled to one another via an adhesive.

The conductor interface 220 is positioned at a proximal end of the substrate 214, and provides a point of electrical contact for the transmission line bundle 112. As described above, the transmission line bundle 112 may comprise a plurality of conductors configured to carry signals to and from the electrical components positioned on the substrate. The conductors of the transmission line bundle 112 are sized, shaped, and otherwise configured to be positioned within the space 266 between the proximal outer member 254 and the proximal inner member 256.

As described above, space available within the spaces provided in the elongate body of the catheter (e.g., within the proximal outer member 254) may be limited. One approach to positioning the conductors of the transmission line bundle 112 within the limited spaces of the catheter is to use a single group of small-gauge wires or ribbons spanning an entire length of the catheter from the scanner assembly to the PIM. The conductors of the bundle 112 may be bundled together to form one or more twisted pairs, twisted quads, twisted groups, or other arrangements of conductors. In some embodiments, one or more of the conductors is non-twisted, such that it runs parallel with one or more conductors or twisted groups of conductors.

It will be understood that, while the embodiments described below include IVUS imaging catheters, the present disclosure contemplates that the described structural features and/or arrangements may be used in other types of intraluminal devices, including sensing catheters, guide catheters, imaging probes, sensing probes, or any other suitable type of device.

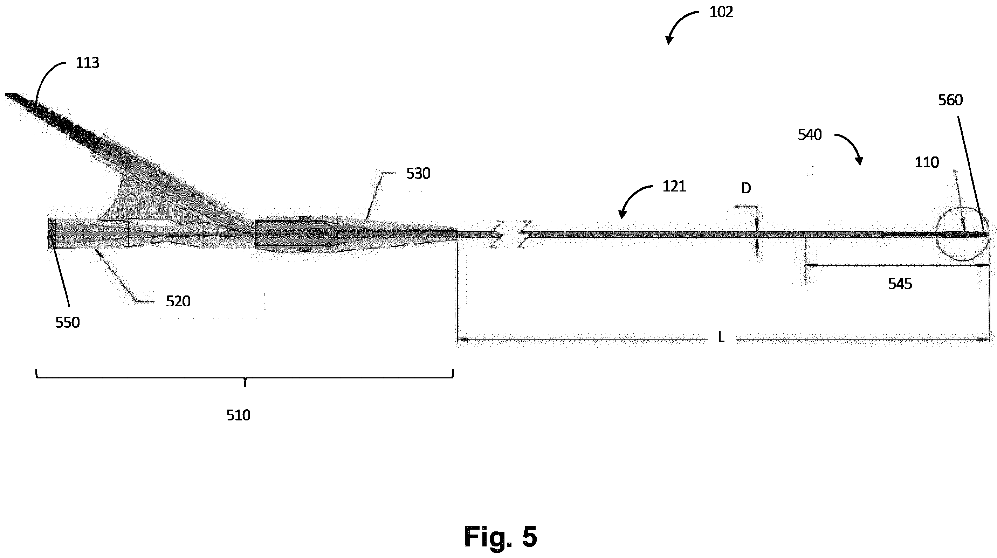

**Fig. 5** is a diagrammatic side view of an intraluminal imaging device 102, in accordance with at least one embodiment of the present disclosure. The device 102 includes a flexible elongate member 121, which includes a working length L (e.g., 150 centimeters, although other working lengths, both longer and shorter, may be used instead or in addition) and a gauge or diameter D (e.g., 5 French, although other diameters both larger and smaller may be used instead or in addition). The flexible elongate member includes a distal portion 540, a hydrophilic coating 545, and a scanner assembly 110. The device 102 also includes a proximal assembly 510 that includes a Y-connector 520. The Y-connector 520 includes a strain relief 530, and a guidewire lumen exit port 550 from which the guidewire 118 (as shown for example in Fig. 1) can emerge after being inserted into a guidewire lumen entry port 560 (located in the distal portion 540 of the flexible elongate member 121) and threaded through the guidewire lumen 236 (as shown for example in Fig. 4). The Y-connector 520 also includes the cable 113, which connects the device 102 to a Patient Interface Module 104 (as shown for example in Fig. 1a).

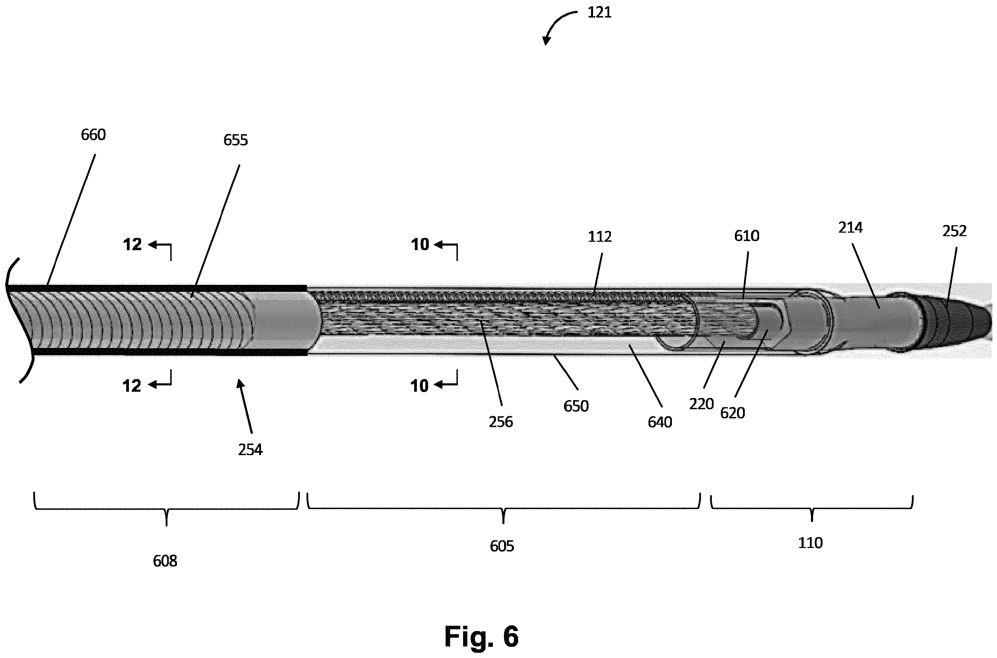

**Fig. 6** is a diagrammatic, cross-sectional side view of an example flexible elongate member 121, in accordance with at least one embodiment of the present disclosure. The flexible elongate member includes a distal member or flexible tip 252 and a scanner assembly 110. Proximal of the scanner assembly 110 is a region 605 (e.g., a more flexible region to assist navigation of the flexible elongate member)) and a region 608 (e.g., a stiffer region to assist pushing of the flexible elongate member).

The scanner assembly 110 includes a flexible substrate or flex circuit 214, a weld leg or conductor interface 220, a sleeve or tube 610 (e.g., made of a polymer such as polyimide, polyamide, thermoplastic elastomer like PEBAX^{®}, or PTFE), a tube extension or guidewire lumen extension 620 (e.g., made of a polymer such as polyimide, polyamide, thermoplastic elastomer like PEBAX^{®}, or PTFE), and a microcable 112 comprising conductors, signal paths, or communication lines 218 (as shown for example in Fig. 2), at least some of which are welded to one or more weld legs 220. It should be understood that signal paths or communication lines 218 may be electrical conductors, fiber optic lines, or other types of signal paths. In that regard, the scanner assembly 110 can include acoustic imaging elements, optical imaging elements, photoacoustic imaging elements, etc. For example, scanner assembly 100 can be an IVUS imaging assembly, intracardiac echocardiography (ICE) imaging assembly, intravascular photoacoustic (IVPA) imaging assembly, optical coherence tomography (OCT) imaging assembly, etc.

In an example, the polymer tube 610 and tube extension 620 help to support and protect the weld leg 220, microcable 112, and the junctions therebetween, and/or to provide electrical insulation to the weld leg 220 (e.g., conductive traces of the weld leg), the conductors 218, and any welds or joins between the weld leg and the conductors.

A portion of the polymer protection tube 610 may also extend into region 605. The polymer tube 610 can be any suitable length, and the proximal and distal ends of the polymer tube can be positioned in a variety of locations. For example, a distal end of the tube 610 can extend partially or completely (longitudinally) over the integrated circuit region 208 of the scanner assembly 110, or completely over the transition region 210 of the scanner assembly 110, partially or completely over the transducer array 112 of the scanner assembly 110. A proximal end of the tube 610 can extend into region 605 of the flexible elongate member 121. The flexible substrate 214 may extend partially or completely along the length of the tube extension or guidewire lumen extension 620 on the proximal side of support member 230.

Region 605 includes an inner member 256 (e.g., composed at least partly of braided metal wires), the microcable 112, and an outer layer 650 (e.g., a flexible cylindrical tube of a polymer such as polyamide like Vestamid^{®}), at least a portion of which is filled with a flexible adhesive 640. In an example, the outer diameter of region 605 is the same as, and is defined by, the outer diameter of the outer layer 650. Depending on the implementation, the durometer or hardness of the flexible adhesive 640 may be selected to adjust the flexibility of region 605, while also enhancing the resistance of region 605 to kinking or buckling as the flexible elongate member is pushed through the tortuous pathways of human vasculature. In some embodiments, portions of the inner member 256, adhesive 640, and outer layer 650 may also extend into the scanner assembly 110.

Region 608 includes the inner member 256, microcable 112, and an outer member 254. In an example, the outer member 254 comprises a tube or shaft 655 composed of metal wires or ribbons enclosed in or covered by a smooth coating or sheath 660 of a biocompatible polymer (e.g., polyimide, polyamide, thermoplastic elastomer like PEBAX^{®}, or PTFE). The metal wires or ribbons can be segments of a metal tube or shaft 655 that are cut in a spiral configuration comprising one, two, three, or more spirals. In other embodiments, the metal wires or ribbons may be embedded in or surrounded by the polymer 660, or else outer member 254 may be composed entirely of polymer or entirely of metal, or of other materials, depending on the implementation. In an example, the outer member 254 is flexible enough to navigate the tortuous pathways of human vasculature, but stiff enough, and resistant enough to buckling or kinking, that the flexible elongate member 121 may be readily pushed forward through the tortuous pathways. In an example, the outer diameter of region 608 is equal to, and defined by, the outer diameter of the outer member 254. The polymer sheath 660 may for example be thermally bonded to the outer layer 650, forming a smooth surface of constant diameter.

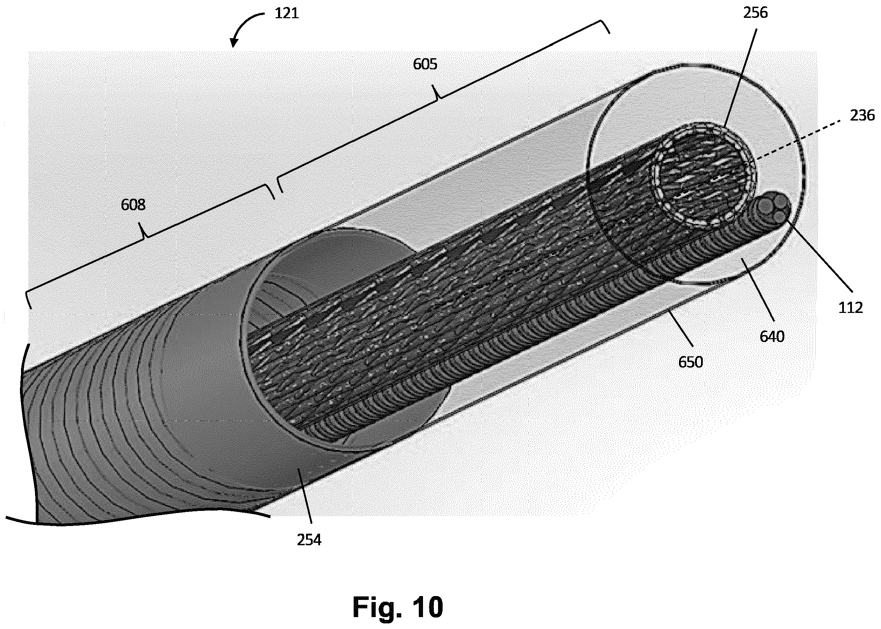

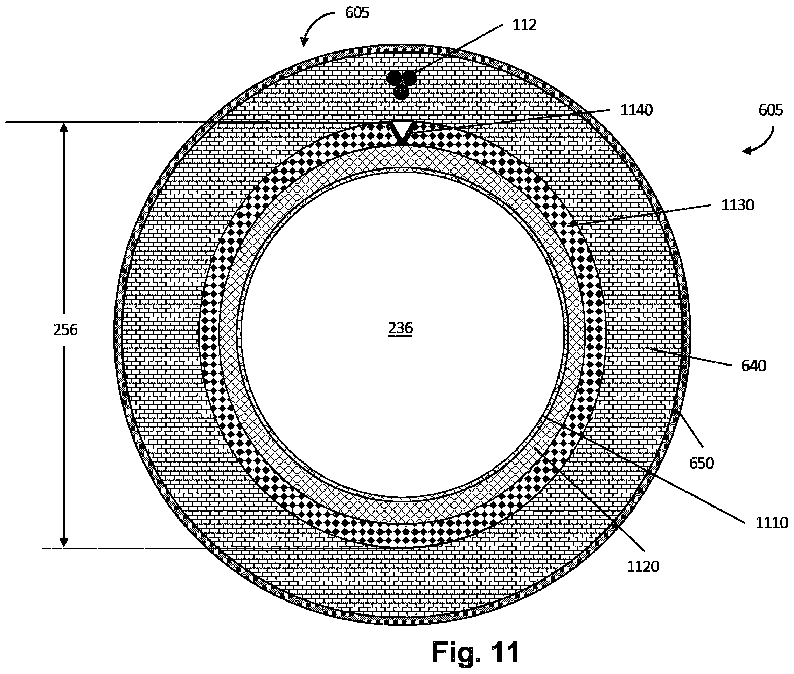

Cut plane 10-10 shows a cross-sectional plane through the flexible elongate member 121, which is shown in greater detail in Fig. 10. Cut plane 11-11 shows a cross-sectional plane through the flexible elongate member 121, which is shown in greater detail in Fig. 11.

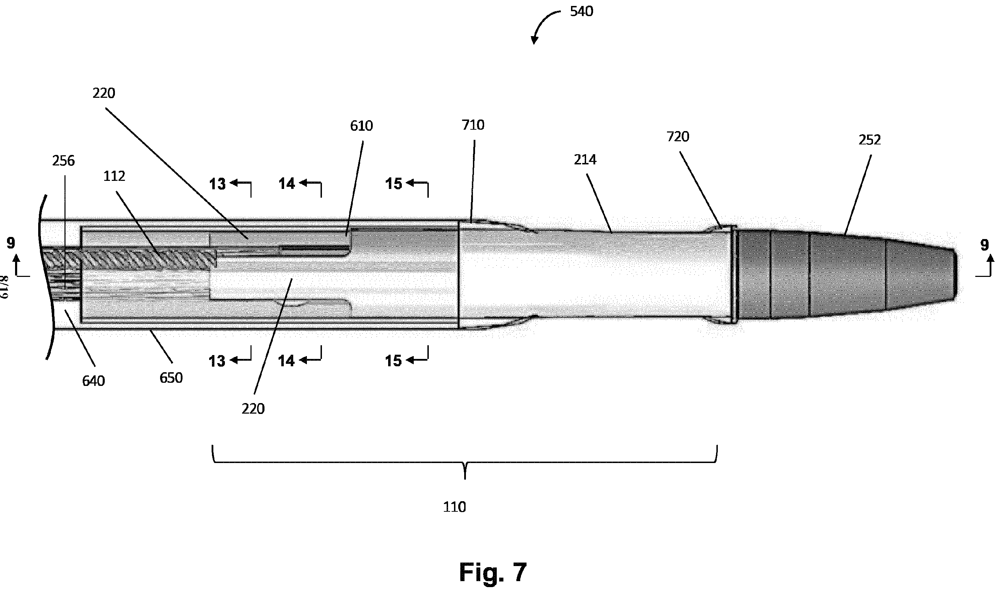

**Fig. 7** is a diagrammatic, cross-sectional side view of a distal portion 540 of an example flexible elongate member 121, in accordance with at least one embodiment of the present disclosure. The distal portion 540 includes the scanner assembly 110 and distal member or flexible tip 252. The scanner portion 110 includes the flexible substrate or flex circuit 214, one or more weld legs or conductor interface 220 (e.g., one, two, three, or more weld legs), the protection sleeve or protection tube 610, the tube extension 620, and the microcable 112. The scanner assembly 110 also includes the inner member 256 and the outer layer 650, at least a portion of which is filled with the flexible adhesive 640. The distal portion 540 also includes adhesive fillets 710 and 720, which may for example form smooth, biocompatible transition regions between the outer surfaces of the flexible substrate 214, outer layer 650, and distal tip 252. In an example, fillets 710 and 720 are formed with a UV-curable adhesive that provides mechanical joint stability and prevents fluid ingress.

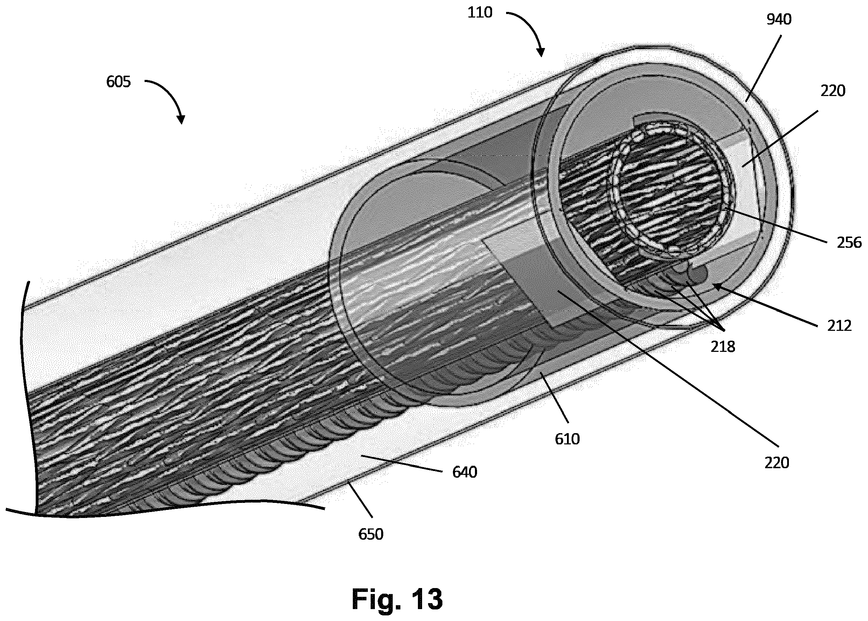

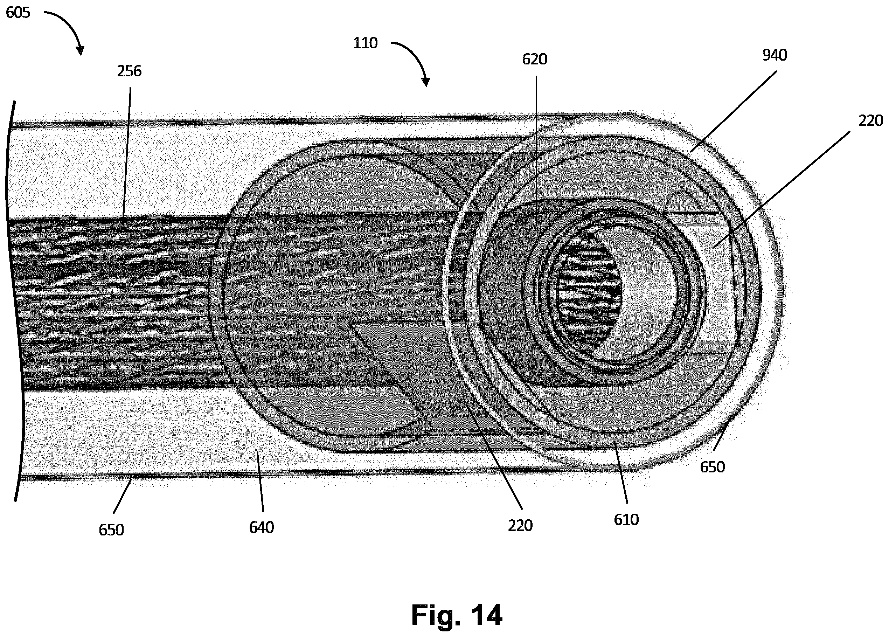

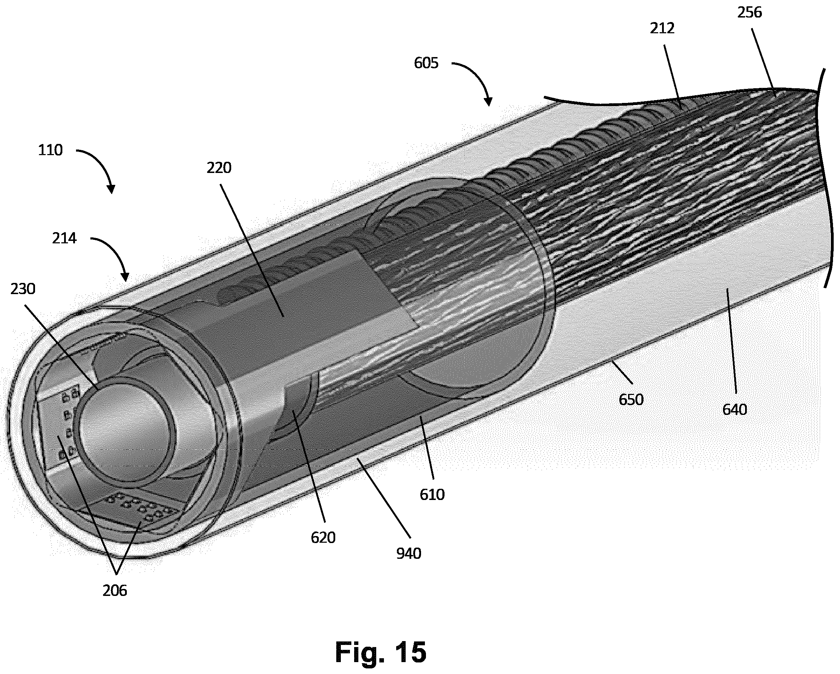

Cut plane 13-13 shows a cross-sectional plane through the distal portion 540 of the flexible elongate member 121, which is shown in greater detail in Fig. 13. Cut plane 14-14 shows a cross-sectional plane through the distal portion 540 of the flexible elongate member 121, which is shown in greater detail in Fig. 14. Cut plane 15-15 shows a cross-sectional plane through the distal portion 540 of the flexible elongate member 121, which is shown in greater detail in Fig. 15.

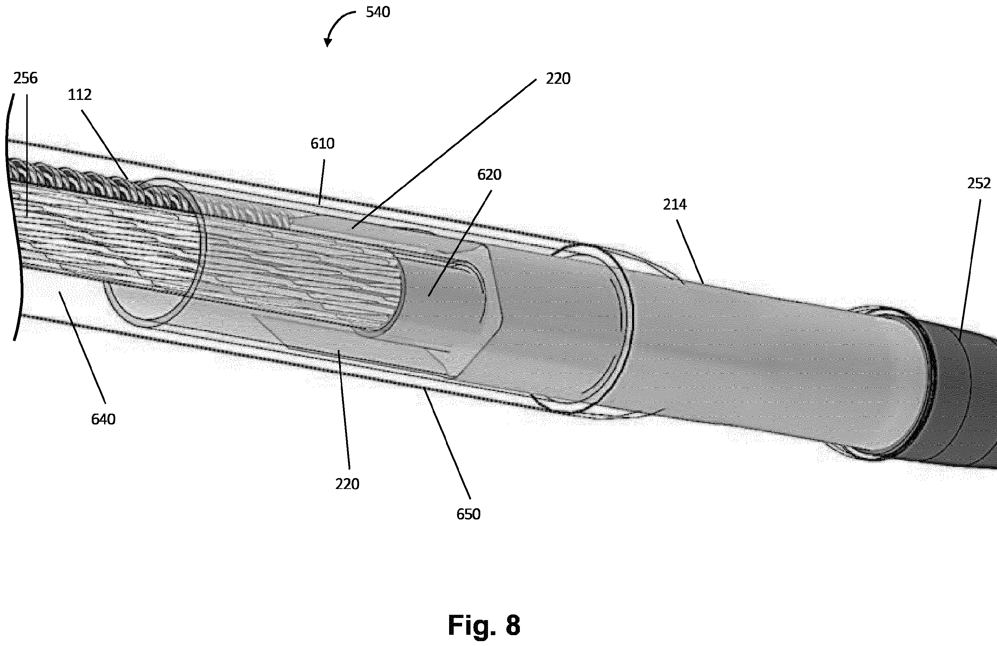

**Fig. 8** is a is a diagrammatic, cross-sectional perspective view of a distal portion 540 of an example flexible elongate member 121, in accordance with at least one embodiment of the present disclosure. The distal portion 540 includes the scanner assembly 110 and distal member or flexible tip 252. The scanner portion 110 includes the flexible substrate or flex circuit 214, one or more weld legs or conductor interfaces 220, the sleeve or tube 610, the tube extension 620, and the microcable 112. The scanner assembly 110 also includes the inner member 256 and the outer layer 650, at least a portion of which is filled with the flexible adhesive 640.

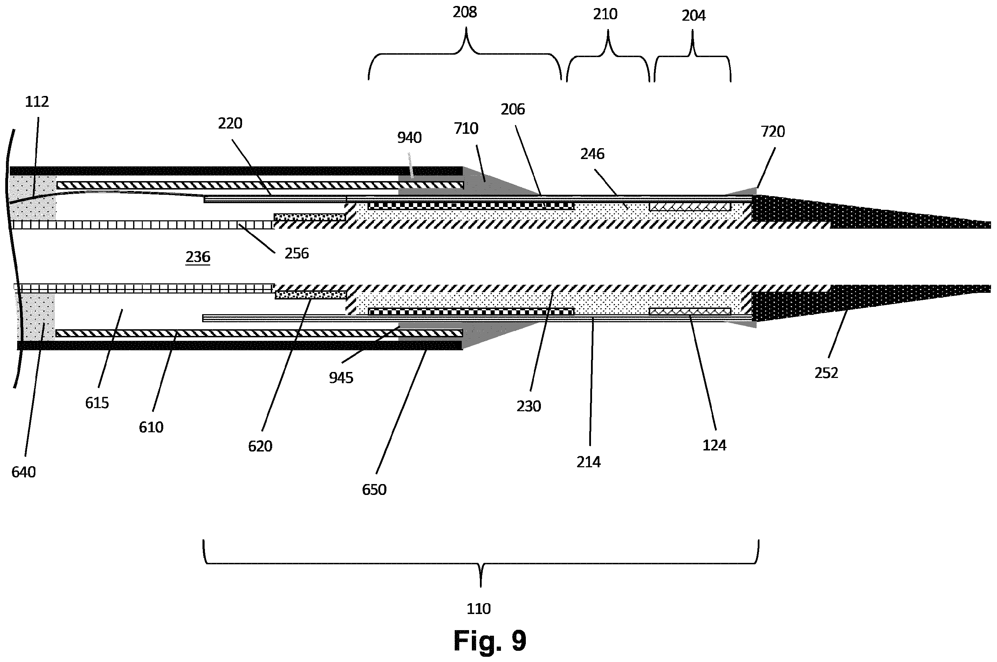

**Fig. 9** is a is a diagrammatic, cross-sectional side view of a scanner assembly 110 of an example flexible elongate member 121, in accordance with at least one embodiment of the present disclosure. In an example, the scanner assembly 110 includes the flexible substrate 214, upon which are formed a transducer array 124 comprising acoustic elements 212 (shown for example in Fig. 2) in a transducer region 204, and transducer control logic dies or controller chips 206 formed in a control region 208, with a transition region 210 disposed therebetween.

In the example shown in Fig. 9, the flexible substrate 214 is wrapped around a support member 230. Cavities between the flexible substrate 214 and the surface of the support member 230 are filled with an acoustic backing material 246 (e.g., an adhesive, electrical insulator, or thermal insulator) to attenuate ultrasound energy propagating in undesired directions (e.g., radially inward). The flexible substrate 214 includes one or more conductor interfaces or weld legs 220, where the conductors 218 of the transmission line bundle or microcable 112 are coupled to the flexible substrate 214. In some embodiments, the support member 230 and/or one or more components thereof may be joined with inner the braided inner member 256 (e.g., by welding, soldering, adhesive, etc.).

The scanner assembly 110 also includes a protection sleeve or protection tube 610 spaced radially outward from the weld legs 220, and a tube extension 620 spaced radially inward from the weld legs 220. In an example, the tube 610 and tube extension 620 help to support and protect the connection between the microcable 112 and the one or more weld legs 220. The microcable 112 may comprise one or more conductors or signal paths 218 (shown for example in Fig. 2). The conductors or signal paths 218 can be twisted or straight, and each conductor 218 can for example be a bare wire surrounded by an insulating layer. In an example, the conductors 218 carry electrical signals from the console and/PIM to the imaging assembly (as shown for example in Fig. 1). Extending longitudinally through the scanner assembly 110 (and in some embodiments, through the entirety of the flexible elongate member), interior to the inner member 256, support member 230, and distal member 252, is a lumen 236, through which the guidewire 118, or other tools or materials, may be threaded or transported.

Spaced radially outward from the inner member 256 is the outer layer 650, with the space between the inner member 256 and the outer member 650 filled with flexible adhesive 640. In some embodiments, the outer layer 650 can be formed of polymer, or metal wires that are braided or spiraled together, or a combination of the two. The polymer can be a coating over the metal wires, or it may be polymer tubing.

In some embodiments, a portion of the flexible adhesive 640 extends between the polymer outer layer 650 and the polymer protection sleeve 610, all the way to adhesive fillet 710. In some embodiments, at least a portion of the space between the outer member 650 and the tube or sleeve 610 is filled with an adhesive 940, which may be the same as, similar to, or different from adhesive 640 or backing material 246. Similarly, at least a portion of the space between the flex circuit 214 (e.g., the weld legs 220) and at least a portion of the polymer sleeve 640 is filled with an adhesive 945, which may or may not be the same ad adhesive 940.

Also visible are adhesive fillets 710 and 720. In an example, adhesive fillets 710 and 720 are positioned on the outer surface of the flexible substrate 214, where they may be directly exposed to the fluids and walls of the vessel 120. Accordingly, they may comprise an adhesive that is smooth, low-friction, and biocompatible. This adhesive may be the same as, similar to, or different from adhesives 640, 940, or 246.

The boundaries between the adhesives need not be straight as shown, but can be angled, irregular, or otherwise, and may be placed differently than shown herein. One adhesive may extend into the other adhesive region and vice versa, or adhesives may partially or completely bond or blend at the interfaces between them.

In some embodiments, space 615 inside the sleeve 610 is not filled (e.g., with adhesive). In such embodiments, the sleeve 610 is thereby advantageously permitted to flex, such as when the imaging device moves through tortuous vasculature. Because the sleeve 610 is coupled to the substrate 214 via the adhesive 710, 940, 945, flexing of the sleeve 610 advantageously causes flexing of the substrate 214 as well.

**Fig. 10** is a diagrammatic, cross-sectional perspective view of cut plane 10-10 of region 605 from Fig. 6, in accordance with at least one embodiment of the present disclosure. Visible are the inner member 256, the interior of which defines lumen 236, and the microcable 112. Inner member 256, lumen 236, and microcable 112 extend through regions 605 and 608. In region 605, an outer layer 650 is spaced radially outward from the inner member 256, and the space between the inner member 256 and outer member 650 is at least partially filled with a flexible adhesive 640, which may or may not also extend into portion 608. In an example, outer layer 650 terminates at the boundary between region 605 and region 608, where it is joined (e.g., by friction fitting, adhesive, or other means) to outer member 254 (which may, for example, be a metal tube that is spiral cut along at least a portion of its length, to render it flexible).

**Fig. 11** is a diagrammatic, cross-sectional view of cut plane 10-10 of region 605 from Fig. 6, in accordance with at least one embodiment of the present disclosure. Visible is the inner member 256, the interior of which defines lumen 236. Also visible are the outer layer 650 (made for example of a polymer such as Vestamid^{®}, polyimide, polyamide, thermoplastic elastomer like PEBAX^{®}, or PTFE), the microcable 112, and the flexible adhesive 640, which fills at least a portion of the space between the inner member 256 and outer layer 650. In an example, the hardness or durometer of the flexible adhesive 640 helps to determine the flexibility of region 605 of the flexible elongate member. The hardness and/or flexibility of the region 605 can be a composite of and/or otherwise result from the individual hardnesses and/ flexibilities of the individual components in the region 605. A particular adhesive 640 (with a given hardness and/or flexibility) can be selected by a manufacturer such that the region 605 has the desired composite hardness and/or flexibility. In that regard, manufacturing is advantageously made more efficient because implementation of a given adhesive 640 and/or changing from one adhesive 640 to another within the region 605 to achieve the desired hardness and/or flexibility may be logistically easier than changing other components within the region 605 to achieve the desired hardness and/or flexibility.

In the example shown in Fig. 11, inner member 256 comprises an inner layer 1110, composed for example of a polymer such as 0.00075" PTFE film, that forms the wall of lumen 236. Inner member 256 further comprises a middle layer 1120 composed for example of mechanically flattened, braided, 0.0007" x 0.003" SS304V wires with a combined ultimate tensile strength of 300,000 to 350,000 PSI, and a braid pitch of 65 ± 5 PPI. Inner member 256 further comprises an interface layer 1130 that may for example be made of a polymer such as 0.001" PEBAX 633 SA01 film. In the example shown in Fig. 11, interface layer 1130 forms the outer surface of inner member 256, and is in circumferential contact with flexible adhesive 640. In some embodiments, the interface layer 1130 may include a slot, notch, or groove 1140 for receiving the microcable 112. Although the microcable 112 is shown fully surrounded by adhesive 640, and not in contact with either the interface layer 1130 or the outer layer 650, it should be noted that the positioning of the microcable 112 may be different than shown here, and may vary over the length of the flexible elongate member, so that for example the microcable 112 may be in contact with either or both of the interface layer 1130 or the outer layer 650.

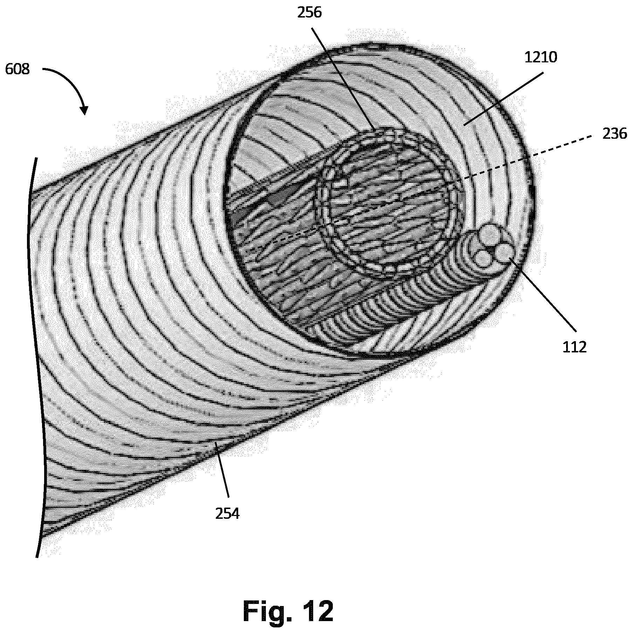

**Fig. 12** is a diagrammatic, cross-sectional perspective view of cut plane 12-12 of region 608 from Fig. 6, in accordance with at least one embodiment of the present disclosure. Visible are the outer member 254 (e.g., a spiral-cut shaft of spring steel coated with an outer layer of polymer), which is hollow and thus defines an outer lumen 1210. Disposed within the outer lumen are the inner member 256 and the microcable 112, which may or may not be bonded to the inner member 256 (e.g., by adhesive, shrink tubing, or other means). The interior of the inner member 256 defines the guidewire lumen or inner lumen 236, through which the guidewire 118 or other tools may be extended.

**Fig. 13** is a diagrammatic, cross-sectional perspective view of cut plane 13-13 of scanner assembly 110 from Fig. 7, in accordance with at least one embodiment of the present disclosure. Visible are portions of the scanner assembly 110 and region 605 of the flexible elongate member 121. Extending through the visible portions of scanner assembly 110 and region 605 are the inner member 256 and microcable 112, which in this example is adhered to an outer surface of the inner member 256 (e.g., with an adhesive). Also visible are the outer layer 650 and the flexible adhesive 640, which fills at least a portion of the space between the inner member 256 and outer layer 650. Additionally visible are the polymer tube or sleeve 610, adhesive 940, and weld legs 220 of the scanner assembly 110, to which conductor 218 of the microcable 112 may be electrically and mechanically coupled (e.g., by welding or soldering).

**Fig. 14** is a diagrammatic, cross-sectional perspective view of cut plane 14-14 of scanner assembly 110 from Fig. 7, in accordance with at least one embodiment of the present disclosure. Visible is the outer layer 650. Flexible adhesive 640 is disposed radially inward from the outer layer 650, and may be in contact with the outer layer 650 along at least a portion of region 605 and at least a portion of scanner assembly 110. Disposed radially inward from flexible adhesive 640 is inner member 256, which may extend through the entirety of region 605 and at least a portion of scanner assembly 110. A tube 610 (e.g., a polymer tube for protection of the weld legs 220) is disposed radially inward from the outer layer 650 and radially outward from the inner member 256, and radially outward from at least a portion of the flexible adhesive 640. A tube extension 620 is disposed radially inward from tube 610 and radially outward from inner member 256, such that at least a portion of tube extension 620 is in circumferential contact with at least a portion of inner member 256. An additional adhesive 940 may be disposed between the tube 610 and the outer layer 650. Also visible are two weld legs 220, although other numbers of weld legs 220 may be employed.

**Fig. 15** is a diagrammatic, cross-sectional perspective view of cut plane 15-15 of scanner assembly 110 from Fig. 7, in accordance with at least one embodiment of the present disclosure. Flexible adhesive 640 is disposed radially inward from the outer layer 650, and may be in contact with the outer layer 650 along at least a portion of region 605 and at least a portion of scanner assembly 110. Disposed radially inward from flexible adhesive 640 is inner member 256. A tube 610 is disposed radially inward from the outer layer 650 and radially outward from inner member 256, and radially outward from at least a portion of the flexible adhesive 640. A tube extension 620 is disposed radially inward from tube 610 and radially outward from inner member 256. Adhesive 940 is disposed between the polymer tube 610 and the outer layer 650. Also visible are microcable 112, support member 230, and various portions of the flex circuit 214, including a weld leg 220 and control chips 206.

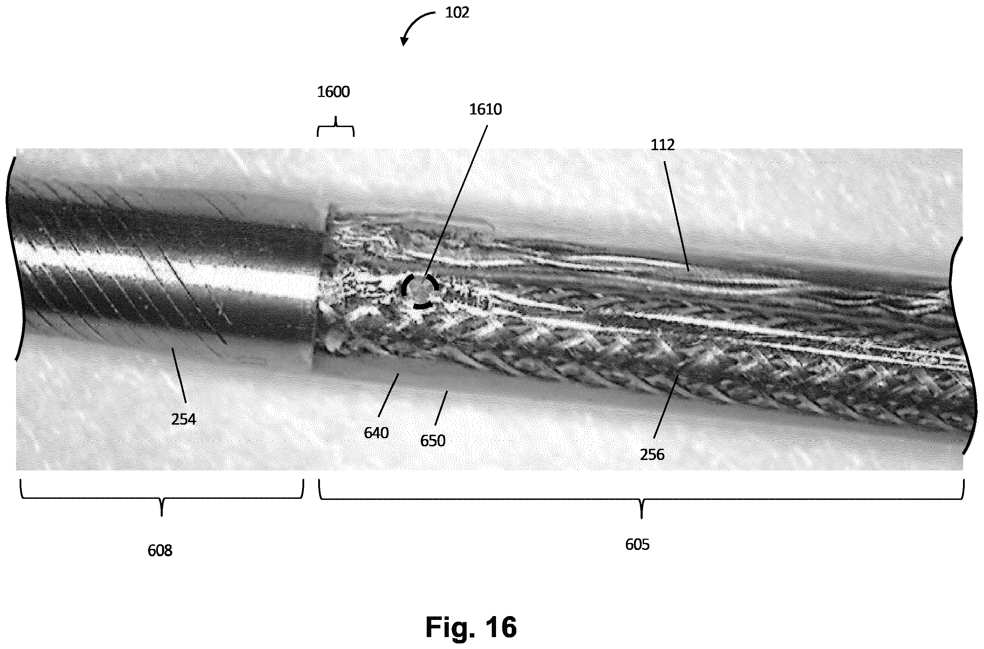

**Fig. 16** is a perspective view of an interface region 1600 between regions 608 and 605 of an example flexible elongate member 121, in accordance with at least one embodiment of the present disclosure. The outer member 254 of region 608 may have a similar or identical diameter to the outer layer 650 of region 605, and may be fixedly coupled to the outer layer 650 of region 605 by a friction fit, an adhesive, or combinations thereof. Visible inside the outer layer 650 is inner member 256, which is surrounded by adhesive 640. In an example, the space between outer layer 650 and inner member 256 is filled with adhesive 640 by means of an aperture 1610 (e.g., a hole drilled or poked into outer layer 650) through which the adhesive 640 is injected in a liquid state (e.g., by a hypodermic needle). Depending on the implementation, after injection the adhesive 640 may harden or be cured, or may remain in a liquid or gel state. Aperture 1610 is then plugged, either by hardening or curing of adhesive 640, or by application and hardening/curing of another adhesive, or by welding of the polymer material of outer layer 650, or by other means.

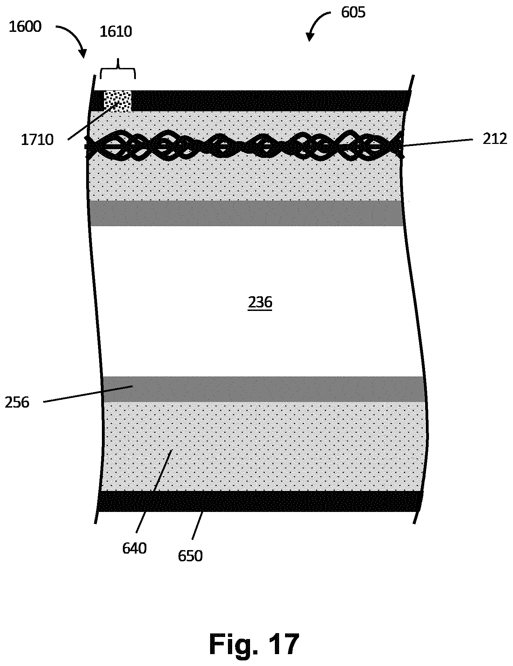

**Fig. 17** is a diagrammatic, cross-sectional side view of the interface region 1600 and region 605 from Fig. 16, in accordance with at least one embodiment of the present disclosure. Visible are the outer member 650, adhesive 640, inner member 256, inner lumen or guidewire lumen 236, microcable 112, and adhesive filling aperture, port, or opening 1610. Within the filling aperture 1610 is a plug 1710, which may comprise the same material as adhesive 640, or may comprise another adhesive, or may comprise the same or a similar material as outer member 650. In an example, plug 1710 is fluorescent, such that it may be examined under ultraviolet light (e.g., using a microscope).

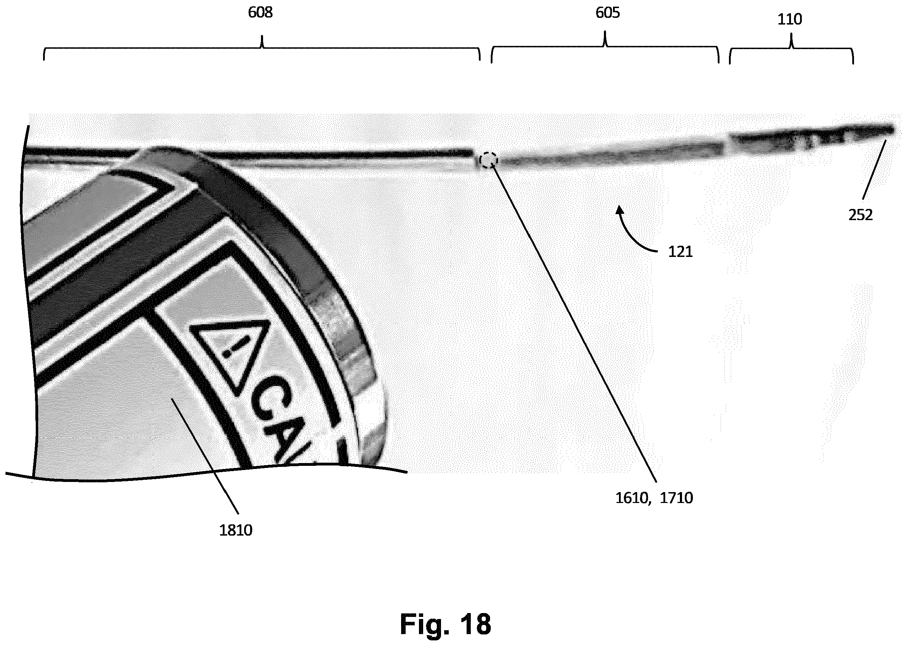

**Fig. 18** is a perspective view of an example flexible elongate member 121, including scanner assembly 110, distal tip 252, region 605, and a portion of region 608, in accordance with at least one embodiment of the present disclosure. Also visible is plug 1710 in aperture or opening 1610. In this example, plug 1710 is fluorescing (e.g., emitting visible light) under the influence of an ultraviolet light source 1810, which may for example be a light-emitting diode, laser diode, or other blue, violet or ultraviolet light source. Fluorescence of the plug 1710 facilitates inspection, such that for example a quality control technician may observe the plug 1710 under a microscope (not pictured) to confirm that aperture 1610 is properly plugged.

A person of ordinary skill in the art will recognize that the present disclosure advantageously provides an intraluminal imaging system that enables both pushability and flexibility for navigating an imaging assembly through human vasculature. The logical operations making up the embodiments of the technology described herein are referred to variously as operations, steps, objects, elements, components, regions, etc. Furthermore, it should be understood that these may occur in any order, unless explicitly claimed otherwise or a specific order is inherently necessitated by the claim language.

It should further be understood that the described technology may be employed in a variety of different applications, including but not limited to human medicine, veterinary medicine, education and inspection. All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the intraluminal imaging system. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure.

## Claims

1. An intraluminal imaging device, comprising:
a flexible elongate member comprising a catheter, the flexible elongate member configured to be positioned within a body lumen of a patient, wherein the flexible elongate member comprises a proximal portion and a distal portion; and
an imaging assembly disposed at the distal portion of the flexible elongate member and configured to obtain intraluminal image data while positioned within the body lumen,
wherein the distal portion of the flexible elongate member comprises a region proximal to the imaging assembly, wherein the region comprises a first hardness that is less than a second hardness of the proximal portion of the flexible elongate member such that the region is more flexible than the proximal portion, wherein the region comprises:
an inner member;
a first adhesive configured to provide the first hardness, wherein the first adhesive surrounds the inner member;
a communication line configured to carry signals associated with the imaging assembly; and
an outer polymer layer surrounding the first adhesive,
**characterized in that** the inner member defines a guidewire lumen, and the communication line is embedded within the first adhesive.

2. The device of claim 1, wherein the outer polymer layer comprises an opening filled with a second adhesive.

3. The device of claim 2, wherein the second adhesive is fluorescent under ultraviolet light.

4. The device of claim 1, wherein the flexible elongate member comprises an outer member proximal to the region, wherein the outer member comprises a metal shaft and a polymer coating surrounding the metal shaft.

5. The device of claim 4, wherein the metal shaft is cut in a spiral configuration.

6. The device of claim 4, wherein the inner member and the communication line extend within the outer member.

7. The device of claim 1, further comprising a polymer tube, wherein a distal portion of the inner member and a proximal portion the imaging assembly are positioned within the polymer tube.

8. The device of claim 7,
wherein the imaging assembly comprises:
a flexible substrate; and
a plurality of integrated circuits disposed on the flexible substrate,
wherein the polymer tube extends over the plurality of integrated circuits.

9. The device of claim 7, further comprising a third adhesive positioned within the polymer tube.

10. The device of claim 1,
wherein the communication line comprises an electrical conductor;
wherein the imaging assembly comprises:
a support member;
a flexible substrate positioned around the support member, wherein the electrical conductor is coupled to a proximal portion of the flexible substrate; and
insulation positioned around the support member and configured to prevent contact between the proximal portion of the flexible substrate and the support member.

11. The device of claim 1, wherein the guidewire lumen extends from the proximal portion of the flexible elongate member to the distal portion of the flexible elongate member.

12. The device of claim 1, wherein the outer polymer layer comprises an outer surface of the flexible elongate member in the region.

13. The device of claim 1, wherein the inner member comprises:
a first polymer layer defining the guidewire lumen;
a wire braid surrounding the first polymer layer; and
a second polymer layer surrounding the first polymer layer.

14. The device of claim 1,
wherein the imaging assembly comprises a circumferential array of acoustic elements, and
wherein the intraluminal image data comprises intravascular ultrasound image data.

15. The device of claim 14,
wherein the inner member comprises:
a first polymer layer defining the guidewire lumen;
a wire braid surrounding the first polymer layer; and
a second polymer layer surrounding the first polymer layer;
wherein the communication line comprises a plurality of electrical conductors configured for communication with the circumferential array of acoustic elements and wherein the outer polymer layer forms an outer surface of the flexible elongate member in the region.

## Patentansprüche

1. Intraluminale Bildgebungsvorrichtung, umfassend:
ein flexibles längliches Element, das einen Katheter umfasst, wobei das flexible längliche Element konfiguriert ist, um in einem Körperlumen eines Patienten positioniert zu werden, wobei das flexible längliche Element einen proximalen Abschnitt und einen distalen Abschnitt umfasst; und
eine Bildgebungsanordnung, die am distalen Abschnitt des flexiblen länglichen Elements angeordnet ist und konfiguriert ist, um intraluminale Bilddaten zu erhalten, während sie im Körperlumen positioniert ist; und
wobei der distale Abschnitt des flexiblen länglichen Elements einen Bereich proximal zur Bildgebungsanordnung umfasst, wobei der Bereich eine erste Härte umfasst, die geringer ist als eine zweite Härte des proximalen Abschnitts des flexiblen länglichen Elements, sodass der Bereich flexibler ist als der proximale Abschnitt, wobei der Bereich umfasst:
ein inneres Element;
einen ersten Klebstoff, der konfiguriert ist, um die erste Härte bereitzustellen, wobei der erste Klebstoff das innere Element umgibt;
eine Kommunikationsleitung, die konfiguriert ist, um Signale zu übertragen, die mit der Bildgebungsanordnung verknüpft sind; und
eine äußere Polymerschicht, die den ersten Klebstoff umgibt,
**dadurch gekennzeichnet, dass** das innere Element ein Führungsdrahtlumen definiert und die Kommunikationsleitung in dem ersten Klebstoff eingebettet ist.

2. Vorrichtung nach Anspruch 1, wobei die äußere Polymerschicht eine mit einem zweiten Klebstoff gefüllte Öffnung umfasst.

3. Vorrichtung nach Anspruch 2, wobei der zweite Klebstoff unter ultraviolettem Licht fluoreszierend ist.

4. Vorrichtung nach Anspruch 1, wobei das flexible längliche Element ein äußeres Element proximal zu dem Bereich umfasst, wobei das äußere Element einen Metallschaft und eine den Metallschaft umgebende Polymerbeschichtung umfasst.

5. Vorrichtung nach Anspruch 4, wobei der Metallschaft in einer Spiralkonfiguration geschnitten ist.

6. Vorrichtung nach Anspruch 4, wobei sich das innere Element und die Kommunikationsleitung innerhalb des äußeren Elements erstrecken.

7. Vorrichtung nach Anspruch 1, die weiter eine Polymerröhre umfasst, wobei ein distaler Abschnitt des inneren Elements und ein proximaler Abschnitt der Bildgebungsanordnung innerhalb der Polymerröhre positioniert sind.

8. Vorrichtung nach Anspruch 7,
wobei die Bildgebungsanordnung umfasst:
ein flexibles Substrat; und
eine Vielzahl integrierter Schaltkreise, die am flexiblen Substrat angeordnet sind, wobei sich die Polymerröhre über die Vielzahl integrierter Schaltkreise erstreckt.

9. Vorrichtung nach Anspruch 7, die weiter einen dritten Klebstoff umfasst, der innerhalb der Polymerröhre positioniert ist.

10. Vorrichtung nach Anspruch 1,
wobei die Kommunikationsleitung einen elektrischen Leiter umfasst;
wobei die Bildgebungsanordnung umfasst:
ein Trägerelement;
ein flexibles Substrat, das um das Trägerelement herum positioniert ist, wobei der elektrische Leiter mit einem proximalen Abschnitt des flexiblen Substrats gekoppelt ist; und
Isolierung, die um das Trägerelement herum positioniert und konfiguriert ist, um Kontakt zwischen dem proximalen Abschnitt des flexiblen Substrats und dem Trägerelement zu verhindern.

11. Vorrichtung nach Anspruch 1, wobei sich das Führungsdrahtlumen vom proximalen Abschnitt des flexiblen länglichen Elements zum distalen Abschnitt des flexiblen länglichen Elements erstreckt.

12. Vorrichtung nach Anspruch 1, wobei die äußere Polymerschicht eine Außenoberfläche des flexiblen länglichen Elements in dem Bereich umfasst.

13. Vorrichtung nach Anspruch 1, wobei das innere Element umfasst:
eine erste Polymerschicht, die das Führungsdrahtlumen definiert;
ein Drahtgeflecht, das die erste Polymerschicht umgibt; und
eine zweite Polymerschicht, welche die erste Polymerschicht umgibt.

14. Vorrichtung nach Anspruch 1,
wobei die Bildgebungsanordnung eine umlaufende Anordnung akustischer Elemente umfasst, und
wobei die intraluminalen Bilddaten intravaskuläre Ultraschallbilddaten umfassen.

15. Vorrichtung nach Anspruch 14,
wobei das innere Element umfasst:
eine erste Polymerschicht, die das Führungsdrahtlumen definiert;
ein Drahtgeflecht, das die erste Polymerschicht umgibt; und
eine zweite Polymerschicht, welche die erste Polymerschicht umgibt;
wobei die Kommunikationsleitung eine Vielzahl elektrischer Leiter umfasst, die zur Kommunikation mit der umlaufenden Anordnung akustischer Elemente konfiguriert sind, und wobei die äußere Polymerschicht eine Außenoberfläche des flexiblen länglichen Elements in dem Bereich bildet.

## Revendications

1. Dispositif d'imagerie intraluminale, comprenant :
un élément allongé flexible comprenant un cathéter, l'élément allongé flexible étant configuré pour être positionné à l'intérieur d'une lumière corporelle d'un patient, dans lequel l'élément allongé flexible comprend une partie proximale et une partie distale ;
un ensemble d'imagerie disposé au niveau de la partie distale de l'élément allongé flexible et configuré pour obtenir des données d'image intraluminale pendant qu'il est positionné à l'intérieur de la lumière corporelle,
dans lequel la partie distale de l'élément allongé flexible comprend une région proximale à l'ensemble d'imagerie, dans lequel la région comprend une première dureté qui est inférieure à une seconde dureté de la partie proximale de l'élément allongé flexible de telle sorte que la région soit plus flexible que la partie proximale, dans lequel la région comprend :
un élément interne ;
un premier adhésif configuré pour fournir la première dureté, dans lequel le premier adhésif entoure l'élément interne ;
une ligne de communication configurée pour transporter des signaux associés à l'ensemble d'imagerie ; et
une couche polymère externe entourant le premier adhésif,
**caractérisé en ce que** l'élément interne définit une lumière de fil-guide et la ligne de communication est intégrée dans le premier adhésif.

2. Dispositif selon la revendication 1, dans lequel la couche polymère externe comprend une ouverture remplie d'un deuxième adhésif.

3. Dispositif selon la revendication 2, dans lequel le deuxième adhésif est fluorescent sous lumière ultraviolette.

4. Dispositif selon la revendication 1, dans lequel l'élément allongé flexible comprend un élément externe proximal à la région, dans lequel l'élément externe comprend une tige métallique et un revêtement polymère entourant la tige métallique.

5. Dispositif selon la revendication 4, dans lequel la tige métallique est coupée dans une configuration en spirale.

6. Dispositif selon la revendication 4, dans lequel l'élément interne et la ligne de communication s'étendent à l'intérieur de l'élément externe.

7. Dispositif selon la revendication 1, comprenant en outre un tube polymère, dans lequel une partie distale de l'élément interne et une partie proximale de l'ensemble d'imagerie sont positionnées à l'intérieur du tube polymère.

8. Dispositif selon la revendication 7,
dans lequel l'ensemble d'imagerie comprend :
un substrat flexible ; et
une pluralité de circuits intégrés disposés sur le substrat flexible, dans lequel le tube polymère s'étend sur la pluralité de circuits intégrés.

9. Dispositif selon la revendication 7, comprenant en outre un troisième adhésif positionné à l'intérieur du tube polymère.

10. Dispositif selon la revendication 1,
dans lequel la ligne de communication comprend un conducteur électrique ;
dans lequel l'ensemble d'imagerie comprend :
un élément de support ;
un substrat flexible positionné autour de l'élément de support, dans lequel le conducteur électrique est couplé à une partie proximale du substrat flexible ; et
une isolation positionnée autour de l'élément de support et configurée pour empêcher un contact entre la partie proximale du substrat flexible et l'élément de support.

11. Dispositif selon la revendication 1, dans lequel la lumière de fil-guide s'étend de la partie proximale de l'élément allongé flexible à la partie distale de l'élément allongé flexible.

12. Dispositif selon la revendication 1, dans lequel la couche polymère externe comprend une surface externe de l'élément allongé flexible dans la région.

13. Dispositif selon la revendication 1, dans lequel l'élément interne comprend :
une première couche polymère définissant la lumière de fil-guide ;
une tresse métallique entourant la première couche polymère ; et
une seconde couche polymère entourant la première couche polymère.

14. Dispositif selon la revendication 1,
dans lequel l'ensemble d'imagerie comprend un réseau circonférentiel d'éléments acoustiques, et
dans lequel les données d'image intraluminale comprennent des données d'image ultrasonore intravasculaire.

15. Dispositif selon la revendication 14,
dans lequel l'élément interne comprend :
une première couche polymère définissant la lumière de fil-guide ;
une tresse métallique entourant la première couche polymère ; et
une seconde couche polymère entourant la première couche polymère ;
dans lequel la ligne de communication comprend une pluralité de conducteurs électriques configurés pour communiquer avec le réseau circonférentiel d'éléments acoustiques et dans lequel la couche polymère externe forme une surface externe de l'élément allongé flexible dans la région.
